# FASCICULE DE BREVET EUROPEEN

(11) **EP 1 537 826 B1**
(45) Date de publication et mention de la délivrance du brevet: **10.11.2010**
(21) Numéro de dépôt: 03292945.7
(22) Date de dépôt: 26.11.2003
(51) Int. Cl.: A61B 6/14, G06T 5/40, H05G 1/28

(54) **Procédé de traitement de signal dans un appareil de radiologie dentaire**
Verfahren zur Signalverarbeitung in einem Dental-Radiologiegerät
Method of signal processing in a dental radiologic apparatus

(43) Date de publication de la demande: 08.06.2005
(73) Titulaire: Carestream Health, Inc., Rochester, New York 14608 (US)
(72) Inventeur: Inglese, Jean-Marc, 77437 Bussy-Saint-Georges (FR)
(74) Mandataire: Santarelli

(56) Documents cités:
- EP-A- 0 224 956
- EP-A- 0 639 819
- EP-A- 0 772 158
- FR-A- 2 750 821
- US-A- 5 663 998
- US-A- 6 047 043
- US-B1- 6 359 628

## Description

L'invention concerne le domaine de la radiologie dentaire.

On connaît des équipements de radiologie dentaire tels que celui décrit dans les brevets français n° 2 547 495 et européen n° 0 129 451.

De tels équipements comprennent une source de rayonnement X qui émet un rayonnement dirigé sur une dent située dans la bouche d'un patient et derrière laquelle se trouve un capteur intrabuccal qui reçoit le rayonnement ayant irradié la dent.

Ce capteur comporte :
- un scintillateur en entrée pour convertir le rayonnement X ayant irradié la dent en rayonnement visible,
- une plaque de fibres optiques pour transmettre le rayonnement visible converti à un détecteur à transfert de charge de type CCD, qui transforme le rayonnement visible converti en un signal électrique analogique, tout en absorbant le résidu de rayonnement X qui n'a pas été converti en rayonnement visible.

Le signal électrique est amplifié et transmis sous forme analogique à travers un long câble, jusqu'à un poste éloigné de traitement et de visualisation où le signal est numérisé et traité afin de produire une image qui est ensuite visualisée sur un écran.

On connaît également, d'après le brevet US 5,912,942, un type de détecteur de rayonnement X dans lequel le détecteur dit à pixels actifs et connu sous le nom de détecteur APS ("Active Pixel Sensor") utilise la technologie de fabrication CMOS.

Dans le brevet précité, l'équipement de radiologie qui y est décrit comprend :
- une source de rayonnement X irradiant un objet,
- un scintillateur qui convertit en lumière visible le rayonnement ayant irradié l'objet,
- éventuellement une plaque de fibres optiques transmettant la lumière visible convertie jusqu'à une matrice à pixels actifs (détecteur) qui la transforme en signal électrique analogique.

Dans ce brevet, il est expliqué que le signal peut être converti en signal numérique dans le capteur lui-même ou dans une boîte d'interface séparée du capteur et distante de l'unité de traitement et de visualisation du signal d'image.

Dans les équipements de radiologique dentaire qui viennent d'être décrits, on ne sait pas si la dose de rayonnement X utilisée pour irradier la dent d'un patient est suffisante pour produire à l'écran de visualisation une image de bonne qualité (niveau d'exposition correct), si la dose est insuffisante (sous-exposition) ou bien excessive (surexposition).

Dans certains cas, un utilisateur particulièrement expérimenté peut, en étudiant l'image de la dent sur l'écran, s'apercevoir que le patient a été exposé à une dose de rayonnement X trop élevée et donc procéder à un réglage approprié de son équipement pour les prises d'image à venir.

Cependant, ceci n'est pas à la portée de la majorité des utilisateurs qui ne savent pas toujours analyser de manière fiable l'image de la dent.

Le document US-A-5663998 décrit un appareil de radiologie dentaire comprenant un capteur intrabuccal et un procédé de traitement du signal avec une étape de détermination dans le signal numérique des valeurs d'amplitude extrêmes.

Le document EP-A-0639819 décrit un appareil de radiologie permettant à l'utilisateur de l'appareil d'adapter le niveau d'exposition pour les images ultérieures en lui fournissant un état d'indication du niveau d'exposition.

Le document FR-A-2750821 explique la classification d'une image de radiologie dentaire en "sur-exposée", "sous-exposée" ou "optimale" selon la largeur et la position de la distribution d'un histogramme.

Compte tenu de ce qui précède, il serait par conséquent utile de pouvoir contrôler, au moins en partie, les niveaux d'exposition au rayonnement X auxquels sont soumis les patients afin d'éviter que ceux-ci ne soient exposés en permanence à des doses de rayonnement X inadaptées.

A cet effet, l'invention concerne un procédé de traitement de signal et un appareil de radiologie dentaire comme définis dans les revendications.

Ainsi, en effectuant un traitement du signal de sortie numérique, on est en mesure de fournir à l'utilisateur de l'appareil de radiologie dentaire selon l'invention un état d'indication du niveau d'exposition au rayonnement X auquel vient d'être soumis le patient, afin d'indiquer s'il s'agit d'un niveau d'exposition insuffisant, satisfaisant ou excessif.

En fonction de l'état d'indication du niveau d'exposition utilisé, il est ainsi possible d'adapter le niveau d'exposition du capteur au rayonnement X pour les signaux de sortie analogiques d'image ultérieurs.

L'invention permet ainsi de rendre l'appareil de radiologie dentaire utilisé plus sécurisant pour les patients qu'auparavant dans la mesure où l'on détecte plus facilement qu'auparavant des doses élevées de rayonnement X auxquelles sont soumis les patients.

En outre, l'invention permet de régler plus facilement qu'auparavant l'appareil de radiologie dentaire puisque l'utilisateur est automatiquement informé du niveau d'exposition utilisé.

Selon une caractéristique, le traitement du signal de sortie numérique d'image est effectué à partir des valeurs d'amplitude extrêmes dudit signal qui ont été préalablement déterminées, afin d'optimiser la plage utile de variation des données numériques en fonction de la plage de variation effective des données analogiques du signal.

Selon une caractéristique, le traitement comporte les étapes suivantes :
- comparaison d'une valeur d'amplitude extrême ou d'une combinaison des valeurs d'amplitude extrêmes à une ou plusieurs valeurs seuil,
- en fonction du résultat de la ou des comparaisons, fourniture de l'état d'indication du niveau d'exposition utilisé.

En s'assurant que l'amplitude de variation du signal analogique correspond à une plage de variation assez large des données numériques, et donc en adaptant la dose de rayonnement émise, on rend maximale l'amplitude des données numériques, améliorant ainsi le rapport signal sur bruit de la conversion, en limitant le bruit dit de conversion numérique-analogique.

Selon une caractéristique, le traitement comprend préalablement une étape de formation d'une courbe traduisant le nombre de pixels du signal d'image par niveau de gris.

Cette courbe, appelée histogramme de l'image, permet de déterminer la plage de variation des données numériques.

Selon une caractéristique, le traitement comprend une étape de détermination des valeurs d'amplitude extrêmes (max, min) des niveaux de gris de la courbe.

Selon une caractéristique, le traitement comprend une étape de détermination d'une différence Δ = max-min.

Cette différence Δ traduit la plage de variation des données numériques et est suffisante pour déterminer le niveau d'exposition utilisé et, ainsi, fournir à l'utilisateur des informations utiles.

Selon une caractéristique, le traitement comprend une première étape de comparaison de la différence Δ à une première valeur seuil1 .

Selon une caractéristique, le traitement comprend une étape de décision quant à la fourniture de l'état d'indication du niveau d'exposition utilisé ou à la réalisation d'une deuxième étape de comparaison en fonction du résultat de la première étape de comparaison.

Selon une caractéristique, l'état d'indication du niveau d'exposition utilisé correspond à un niveau de sous-exposition lorsque la différence Δ est inférieure ou égale à la première valeur seuil1.

Lorsque le niveau d'exposition aux rayons X est faible, le signal analogique est faible et donc la plage de variation des données numériques est réduite.

Selon une caractéristique, lorsque la différence Δ est supérieure à la première valeur seuil1, alors le traitement comprend une deuxième étape de comparaison de la valeur max à une deuxième valeur seuil2.

Selon une caractéristique, le traitement comprend une étape de décision quant à la fourniture de l'état d'indication du niveau d'exposition utilisé ou à la réalisation d'un test additionnel, en fonction du résultat de la deuxième étape de comparaison.

Selon une caractéristique, l'état d'indication du niveau d'exposition utilisé correspond à un niveau d'exposition correct lorsque la valeur max est inférieure à la deuxième valeur seuil2.

Cette valeur max est représentative des pixels ayant reçu le plus de rayonnement. Lorsque cette valeur se rapproche de la valeur maximale des données numériques, on se rapproche de la valeur maximale de la dose de rayons X qui correspond à la saturation de la chaîne de traitement du signal, et donc de la surexposition de l'image.

Selon une variante, on compare la différence Δ à une valeur seuil3 afin de détecter la surexposition. Cette variante est particulièrement utile sur une image comportant un grand nombre de niveaux de gris et qui, par exemple, a été réalisée sur un objet relativement hétérogène du point de vue des densités radiologiques.

Selon une caractéristique, lorsque la valeur max est supérieure ou égale à la deuxième valeur seuil2, alors le traitement comprend une étape de test additionnel afin de déterminer la présence ou l'absence de données indicatives d'un débordement dans le signal de sortie numérique.

Selon une caractéristique, en fonction de la détermination de la présence ou de l'absence de données indicatives d'un débordement, le traitement comporte une étape de décision quant à la fourniture de l'état d'indication du niveau d'exposition utilisé correspondant respectivement à un niveau de surexposition ou à un niveau d'exposition correct.

Cette étape supplémentaire permet de s'affranchir d'éventuelles variations dans l'amplitude du signal analogique d'un capteur à l'autre. L'amplitude de variation du signal analogique correspond idéalement à l'amplitude de variation des données numériques, appelée fenêtre d'entrée du convertisseur analogique-numérique. Il est souhaitable de s'assurer par conception que l'amplitude de variation du signal analogique ne soit pas inférieure à la fenêtre du convertisseur analogique-numérique, cela afin d'éviter de ne pas tirer parti de la plage des variations des données numériques. Dans le cas inverse où l'amplitude de variation du signal analogique peut, pour un capteur donné, dépasser la fenêtre du convertisseur analogique-numérique, les données indicatives d'un débordement (bit de débordement) fournissent également une indication de saturation de la chaîne de traitement du signal.

Selon une caractéristique, le procédé comporte une étape d'affichage de l'état d'indication du niveau d'exposition utilisé sur un écran de visualisation.

Selon une caractéristique, le procédé comporte une étape d'affichage du signal d'image correspondant sur l'écran de visualisation.

Selon une caractéristique, l'état d'indication du niveau d'exposition est affiché sous la forme d'au moins un indicateur dont la position varie en fonction de l'état obtenu par le traitement du signal et, par exemple, en fonction de la différence Δ obtenue.

Cet indicateur peut se présenter avantageusement, mais non exclusivement, sous la forme d'un curseur linéaire.

Selon une caractéristique, l'état d'indication du niveau de sous-exposition, d'exposition correcte ou de surexposition est affiché sous la forme d'un curseur produisant un effet visuel qui varie au moins pour certains niveaux d'exposition.

On peut ainsi détecter rapidement un niveau d'exposition critique d'un niveau normal.

Par exemple, l'effet visuel est la couleur.

Selon une caractéristique, l'étape de conversion est effectuée dans un convertisseur analogique numérique ayant une fenêtre d'entrée adaptée à la dynamique du signal analogique délivré par le capteur.

D'autres caractéristiques et avantages apparaîtront au cours de la description qui va suivre, donnée uniquement à titre d'exemple non limitatif et faite en référence aux dessins annexés, sur lesquels :
- la figure 1 est une vue schématique générale de l'appareil de radiologie dentaire selon l'invention, lors d'une prise d'image d'une dent ;
- la figure 2 est une vue d'un algorithme d'un programme informatique exécuté dans l'unité centrale de la figure 1 ;
- la figure 3 est une vue schématique illustrant un histogramme traduisant le nombre de pixels du signal d'image en fonction du niveau de gris ;
- la figure 4 illustre trois histogrammes de signaux de sortie d'image correspondant respectivement à trois niveaux d'exposition au rayonnement X du capteur ;
- les figures 5a, 5b, 5c illustrent respectivement trois positions possibles du curseur représenté à la figure 1 pour les différents histogrammes illustrés à la figure 4.

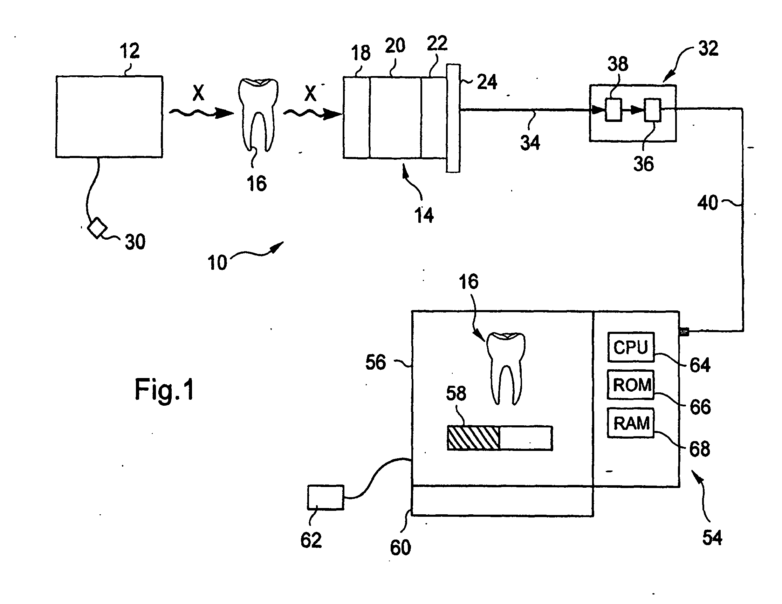

Comme représenté à la **figure 1**, un appareil de radiologie dentaire à rayons X 10 comprend une source de rayonnement X 12 placée à l'extérieur de la bouche d'un patient et un capteur intrabuccal de rayonnement 14 disposé dans la bouche d'un patient, derrière une dent 16, et qui est apte à recevoir le rayonnement X qui irradie la dent.

Le capteur 14 comprend, suivant l'ordre de propagation du rayonnement, un scintillateur 18 qui convertit le rayonnement X ayant irradié la dent en rayonnement visible, éventuellement une plaque de fibres optiques 20 qui, d'une part, comprend des particules métalliques destinées à absorber la partie du rayonnement X reçue par le scintillateur et non convertie en rayonnement visible et, d'autre part, conduit le rayonnement visible ainsi converti jusqu'à un détecteur 22. Ce détecteur est monté sur un substrat 24 en céramique et transforme le rayonnement visible issu des fibres optiques en un ou plusieurs signaux électriques analogiques.

On notera que d'autres structures de capteur peuvent convenir et, par exemple, un capteur avec un scintillateur directement agencé contre le détecteur.

Les différents composants du capteur 14 sont assemblés les uns avec les autres, par exemple, par collage.

Le scintillateur 18 est, par exemple, réalisé en oxysulfure de gadolinium.

Alternativement, il pourrait être réalisé en iodure de Césium, en cristaux de Lutécium ou en tout élément ayant la propriété de transformer le rayonnement X en rayonnement visible.

La plaque de fibres optiques 20 lorsqu'elle est présente est, par exemple, commercialisée par la société SCHOTT sous la référence commerciale 47A ou par la société HAMAMATSU sous la référence commerciale XRS.

Le détecteur 22 peut être un détecteur à transfert de charge (CCD) du type de celui utilisé dans le brevet français FR 2 547 495 ou européen n° 0 129 451.

Ce détecteur 22 peut également, et de façon non limitative, être un détecteur de type APS dit à pixels actifs (connu en terminologie anglosaxonne sous le terme "Active Pixel Sensor") utilisant la technologie de fabrication CMOS, tel que décrit dans le brevet US 5,912,942.

L'appareil comprend également un organe d'activation 30 du générateur de rayonnement 12 afin d'émettre les rayons X et éventuellement d'adapter la dose de rayons. Le réglage de la dose peut toutefois être effectué autrement et, par exemple, directement sur le générateur.

L'appareil de radiologie 10 comprend un module électronique 32 distant du capteur 14 et donc du détecteur 22 de la figure 1, qui est relié au capteur par une liaison filaire 34 qui est un câble.

Par exemple, le câble est de type multifilaire, multibrins, les signaux rapides (horloge, vidéo, ...) pouvant optionnellement faire l'objet de câbles coaxiaux et l'ensemble étant blindé par une tresse de masse.

On notera que le module électronique comprend principalement un convertisseur analogique numérique 36 et un bloc 38 d'amplification et de filtrage avant conversion.

L'appareil de radiologie 10 comporte également une unité de traitement et de visualisation 54 distante du module électronique 32 et reliée à ce dernier par l'intermédiaire d'une liaison filaire 40 qui est, par exemple, un câble.

L'unité de traitement et de visualisation 54 est, par exemple, un ordinateur qui va recevoir les signaux de sortie du capteur 14, une fois numérisés dans le module électronique 32, afin d'effectuer sur ces derniers un traitement d'image approprié et connu de l'homme de l'art pour visualiser sur l'écran 56 l'image de la dent 16 de la figure 1.

On notera que la conversion peut alternativement être directement effectuée dans l'unité 54 et, dans ce cas, on se passe du module 32.

Une interface conforme à la norme USB2.0 est, par exemple, prévue dans le module 32, ainsi qu'un bus série USB2.0 et une interface (non représentée) correspondante dans l'ordinateur distant, pour que les signaux délivrés par le module 32 soient transmis à l'ordinateur distant 54 avec un haut débit, par exemple de l'ordre de 480 Mbits/s.

L'utilisation d'une telle interface permet ainsi de transmettre rapidement à l'ordinateur les données qui sont fournies par le détecteur 22 et traitées par le module 32.

L'utilisation de cette interface convient particulièrement bien à l'utilisation d'un signal d'horloge de fréquence élevée, par exemple à 12 MHz, pour l'échantillonnage des données recueillies par le détecteur 22, ceci sans avoir à utiliser une mémoire tampon pour le stockage des données avant leur transmission à l'ordinateur.

En effet, cette fréquence de signal d'horloge représente un bon compromis entre, d'une part, une fréquence trop basse pour échantillonner le détecteur, ce qui provoquerait une augmentation du courant d'obscurité entre le début et la fin de l'échantillonnage du détecteur et, d'autre part, une fréquence trop élevée qui générerait un bruit de lecture supplémentaire perturbant le signal de sortie du détecteur.

En effet, compte tenu des contraintes liées à l'évolution au cours du temps du courant d'obscurité dans le détecteur, il faut échantillonner les pixels du détecteur à une fréquence relativement élevée qui s'avère incompatible avec le débit de transmission autorisé par la norme USB1.

On notera que la vitesse de transmission des données sur la liaison filaire 40 doit être au moins égale à celle prévue par la norme USB2.0 afin de pouvoir vider rapidement le détecteur (par exemple une matrice de pixels actifs) sans avoir recours à une mémoire tampon.

Il convient de noter que le convertisseur analogique numérique 36 présente une fenêtre d'entrée qui est adaptée à la dynamique du signal analogique de sortie délivré par le capteur 14 et, plus particulièrement par le détecteur 22 afin d'éviter qu'en cas de dimensionnement de fenêtre trop large, le signal attaquant le convertisseur ne puisse jamais saturer ce dernier.

Le dimensionnement d'une telle fenêtre peut être obtenu par une approche successive et par de simples mesures d'exécution à la portée de l'homme du métier.

On choisit, par exemple, un convertisseur qui échantillonne sur 12 bits le signal analogique de sortie qui lui est fourni en entrée et on adopte une fenêtre de largeur d'entrée, par exemple, de 2 volts.

Ceci signifie que pour un signal de 0 volt en entrée on obtient une valeur de 0 en sortie du convertisseur et pour un signal d'entrée de 2 volts, on obtient une valeur de 4095 en sortie.

Si le signal d'entrée présente, par exemple, une tension de 2,1 volts, alors on obtient en sortie du convertisseur une valeur de 4095 ainsi que, dans le signal de sortie numérique, des données (bit) indicatives d'un débordement (connu en terminologie anglosaxonne sous le terme de "overflow") indiquant une saturation.

On notera que des convertisseurs échantillonnant sur un nombre de bits différent peuvent être utilisés dans le cadre de l'invention.

L'unité de traitement et de visualisation 54 comporte comme on l'a vu précédemment l'écran 56 sur lequel est affiché ou visualisé le signal d'image de la dent 16, ainsi qu'au moins un indicateur (curseur ou barre de progression) 58 représentée sur l'écran 56 sous l'image de la dent.

Comme le verra ultérieurement, la position de cet indicateur (par exemple un curseur) est représentative d'un niveau d'exposition du capteur 14 au rayonnement X utilisé (dose reçue par le patient) pour délivrer le signal de sortie analogique considéré.

D'autres indicateurs ou éléments d'interface peuvent, bien entendu, être utilisés pour fournir à l'utilisateur de l'appareil de radiologie dentaire un état d'indication du niveau d'exposition au rayonnement X utilisé.

L'unité 54 comprend également un clavier 60 et une interface d'entrée telle qu'une souris 62.

Par ailleurs, l'unité 54 comprend une unité centrale de traitement 64, une mémoire morte 66 et une mémoire vive 68.

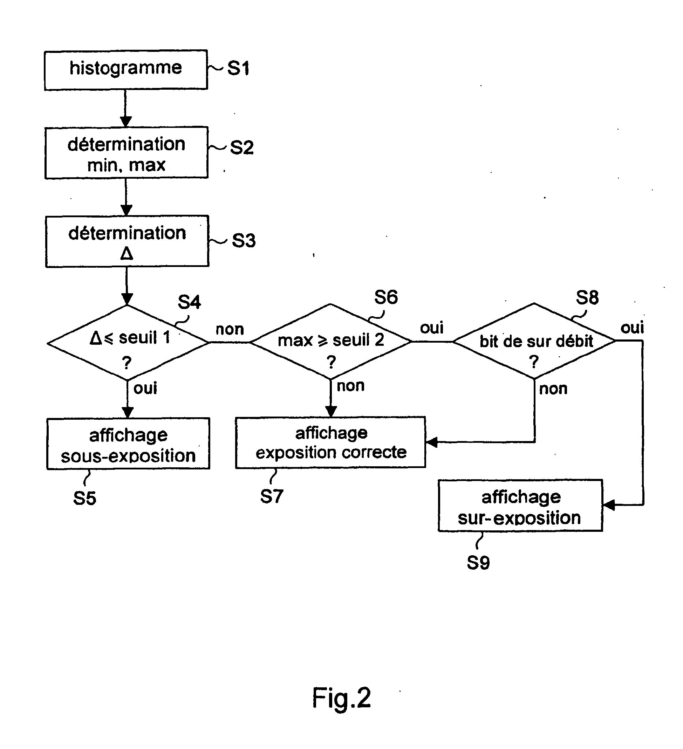

Un programme d'ordinateur dont l'algorithme est représenté sur la **figure 2** est stocké dans la mémoire 66 et, à l'initialisation du système, chargé dans la mémoire 68 puis exécuté par l'unité centrale 64.

L'algorithme de la figure 2 comporte une série d'étapes correspondant à des instructions ou portions de code d'un logiciel qui, une fois exécutées par l'unité 54, vont permettre la mise en oeuvre du procédé selon l'invention.

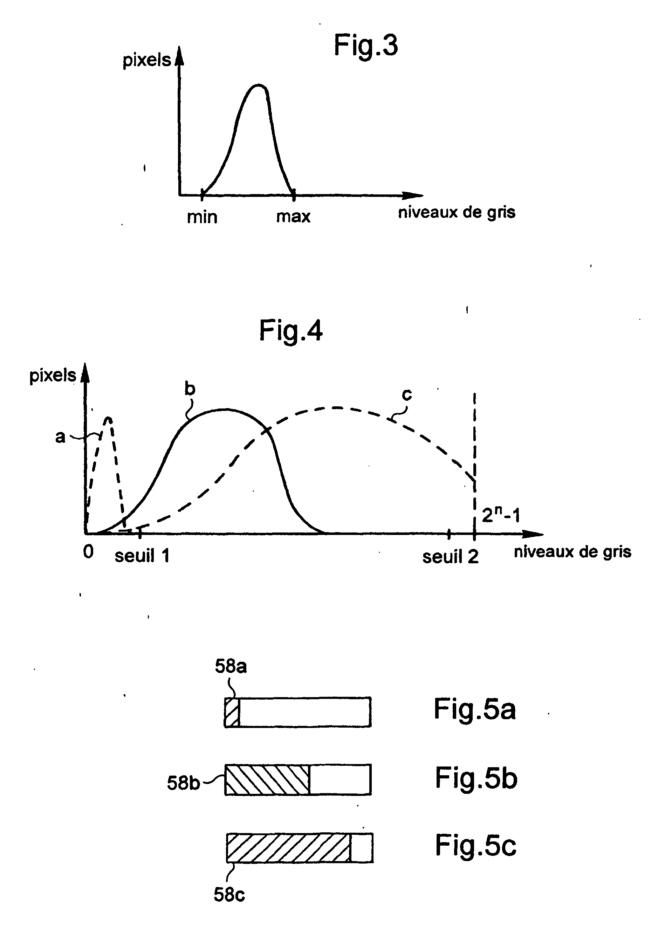

L'algorithme de la figure 2 comporte une première étape S1 au cours de laquelle on détermine l'histogramme du signal de sortie numérique qui traduit, comme représenté à la **figure 3**, le nombre de pixels donné par niveau de gris numérique dans le signal de sortie numérique d'image.

Sur la figure 3 on voit ainsi apparaître pour l'histogramme considéré une valeur d'amplitude extrême de niveau de gris appelée min (valeur minimale) et une valeur d'amplitude extrême de niveau de gris du signal appelée max (valeur maximale).

L'histogramme se traduit sous la forme d'un tableau de 2ⁿ éléments soit 4096 dans l'exemple choisi avec un convertisseur échantillonnant sur 12 bits.

Au cours de l'étape suivante S2, on procède à la détermination dans le signal numérique, et plus particulièrement dans l'histogramme, des valeurs d'amplitude extrêmes du signal (en terme de niveaux de gris dans l'histogramme de la figure 3), à savoir les valeurs min et max précitées.

Pour ce faire, on parcourt le tableau de 4096 éléments, dont la largeur est celle de l'histogramme, afin de déterminer, d'une part, la plus petite valeur non nulle de ce tableau (min) et, d'autre part, la plus grande valeur non nulle de ce tableau (max).

On notera que la réalisation de l'histogramme de la figure 3 correspond à un traitement statistique du signal d'image par niveaux de gris qui est connu de l'homme du métier.

Au cours de l'étape suivante S3, on prévoit de déterminer la différence Δ entre la valeur maximale et la valeur minimale déterminée à l'étape précédente.

A partir de cette différence, on est déjà en mesure de déterminer la position du curseur illustrée sur les figures.

L'algorithme comprend ensuite un première étape S4 de comparaison de la différence Δ à une première valeur de seuil notée seuil1 et qui est, par exemple, fixée à la valeur 256.

Cette valeur est indicative d'une dose de rayonnement X insuffisant (niveau d'exposition qualifié de sous-exposition).

Selon le résultat de cette étape de comparaison, il va être possible de prendre une décision quant à la fourniture de l'état d'indication du niveau d'exposition utilisé lors de la prise d'image ou quant à la réalisation d'une deuxième étape de comparaison

Ainsi, dans le cas où la différence Δ est inférieure ou égale à la valeur seuil1, alors l'étape S4 est suivie d'une étape S5 qui prévoit de fournir à l'utilisateur l'état d'indication du niveau d'exposition utilisé pour la prise d'image concernée.

L'état d'indication du niveau d'exposition utilisé correspond ici à une sous-exposition qui est affichée sur l'écran de visualisation.

La position correspondante du curseur 58 pour un niveau d'exposition insuffisant (sous-exposition) est représentée à la **figure 5a** et est identifiée par une zone de petites dimensions 58a sur la partie gauche.

Lorsque le curseur se trouve dans cette position, l'utilisateur est informé de ce qu'une dose de rayonnement X insuffisante a été utilisée pour prendre l'image de la dent du patient et l'image qui en résulte présente donc une sous-exposition.

Il convient alors d'augmenter la dose de rayonnement X pour la prochaine prise d'image.

Ce phénomène est illustré sur la **figure 4** par l'histogramme référencé a.

On notera que la valeur 2ⁿ-1 représente l'amplitude de la fenêtre de sortie du convertisseur utilisé qui, dans l'exemple décrit, est fixée à 4095 (12 bits de signal).

Lorsque la différence Δ est supérieure à la première valeur seuil1, alors le traitement de signal de l'algorithme de la figure 2 prévoit une deuxième étape de comparaison S6 au cours de laquelle on compare la valeur max à une deuxième valeur seuil2 qui est choisie, par exemple, de manière à être proche de la valeur 2ⁿ-1 de la figure 4. Cette valeur seuil est, par exemple, fixée dans l'exemple considéré à 2ⁿ-1 - seuil1, à savoir ici 4095 - 256.

La comparaison de la valeur max à cette valeur seuil2 proche du bord de la fenêtre de sortie du convertisseur va permettre de fournir une indication sur le niveau d'exposition utilisé.

En effet, lorsque la valeur max est strictement inférieure à la valeur seuil2, alors il est décidé de fournir à l'utilisateur l'état d'indication du niveau d'exposition utilisé qui correspond à une exposition correcte et d'afficher cet état d'indication du niveau d'exposition sur l'écran 56 de la figure 1.

Ce cas de figure correspond à une dose de rayonnement X satisfaisante qui ne va pas nécessiter de régler l'appareil de la figure 1 et plus particulièrement la source de rayonnement X 12 (modification de la dose de rayons X).

Le niveau d'exposition correspondant est indiqué par la position du curseur 58b sur la **figure 5b**.

Pour bien distinguer les positions des deux curseurs respectifs des figures 5a et 5b, on peut, par exemple, représenter le curseur 58a en rouge et le curseur 58b en vert ou utiliser un autre effet visuel permettant de distinguer les deux curseurs l'un de l'autre et donc de différencier les deux niveaux d'exposition.

On notera que la figure 5b fournit sous forme d'affichage l'état d'indication du niveau d'exposition utilisé pour produire un signal de sortie dont l'histogramme est celui référencé b sur la figure 4.

Lorsque la valeur max est supérieure ou égale à la valeur prédéterminée seuil2, alors l'étape S6 est suivie d'une étape S8 au cours de laquelle un test est effectué afin de déterminer si des données indicatives d'un débordement sont présentes dans le signal de sortie numérique fourni par le convertisseur.

Ces données se présentent, par exemple, sous la forme d'un bit de débordement dont la présence est représentative d'un signal d'entrée dont la dynamique est plus grande que celle de la fenêtre d'entrée du convertisseur.

Lorsqu'une telle valeur indicative d'un débordement est absente du signal, alors l'étape S8 est suivie de l'étape S7 précédemment décrite.

Ce cas correspond à un histogramme dont l'allure est celle de l'histogramme référencé b sur la figure 4 et dont l'état d'indication du niveau d'exposition utilisé est illustré sur la figure 5b.

Toutefois, dans ce cas, la valeur max de l'histogramme b est cependant comprise entre la valeur seuil2 et la valeur 2ⁿ-1.

Au contraire, lorsque la présence d'une valeur indicative d'un débordement est détectée dans le signal numérique, alors l'étape S8 est suivie d'une étape S9 qui prévoit de fournir à l'utilisateur l'état d'indication correspondant du niveau d'exposition utilisé.

Ici, le niveau d'exposition utilisé correspond à une surexposition, ce qui signifie que la dose de rayonnement X utilisée pour la prise d'image de la dent visualisée était excessive.

L'état d'indication du niveau d'exposition utilisé à la **figure 5c** par a position du curseur 58c qui occupe la quasi-totalité du rectangle.

On notera que pour distinguer ce cas de celui de la figure 5b, on peut, par exemple, représenter en rouge le curseur 58c, le curseur 58b étant quant à lui représenté en vert, voire utiliser un autre effet visuel (motifs différents du curseur, modification des dimensions du curseur, autre élément apparaissant à l'écran ...).

On peut également utiliser un indicateur sonore en plus ou à la place de l'indicateur visuel.

La position du curseur sur la figure 5c fournit l'état d'indication du niveau d'exposition au rayonnement utilisé pour produire un signal de sortie du capteur dont l'histogramme est référencé c sur la figure 4.

On notera que dans les cas extrêmes représentés par les courbes a et c illustrées aux figures 5a et 5c, la fourniture à l'utilisateur de l'appareil de l'état d'indication du niveau d'exposition correspondant qui est utilisé permet à ce dernier de régler de façon adaptée son appareil.

Ainsi, en cas de sous-exposition, il augmentera la dose de rayonnement pour le prochain patient et, en cas de surexposition, il réduira la dose de rayonnement X.

L'invention permet donc très facilement et de façon automatique, de détecter si l'image est surexposée ou sous-exposée et donc si les doses de rayonnement X utilisées étaient dangereuses ou non pour le patient sans avoir besoin de procéder à un examen de l'image de la dent produite sur l'écran 56.

En effet, l'information sur l'état d'indication du niveau d'exposition utilisé qui est fournie à l'utilisateur est particulièrement utile à ce dernier qui ne sait pas toujours déterminer le niveau d'exposition en visualisant l'image de la dent sur l'écran.

Il convient de remarquer que l'algorithme de la figure 2 pourrait être complété, par exemple, en ajoutant des étapes de comparaison par rapport à d'autres valeurs seuil afin d'affiner l'information fournie à l'utilisateur.

L'utilisateur gagne également un temps précieux en disposant d'une information non ambiguë et non susceptible d'erreur de lecture, ce qui rend l'invention particulièrement efficace et l'appareil fiable.

On notera également que l'affichage de l'état d'indication du niveau d'exposition utilisé pour produire un signal d'image d'une dent peut être réalisé en même temps que l'affichage du signal d'image lui-même comme illustré sur la figure 1.

## Revendications

1. Procédé de traitement de signal dans un appareil de radiologie dentaire comprenant un capteur intrabuccal qui délivre au moins un signal de sortie analogique d'image en réponse à une exposition dudit capteur à un rayonnement X, comportant les étapes suivantes:
- conversion dudit au moins un signal de sortie analogique d'image en un signal de sortie numérique d'image avec un convertisseur ayant une fenêtre de sortie,
- traitement du signal de sortie numérique d'image en vue d'obtenir un état d'indication du niveau d'exposition au rayonnement X qui a été utilisé pour délivrer ledit signal de sortie analogique, l'état d'indication du niveau d'exposition correspondant à un niveau d'exposition qualifié de sous-exposition, d'exposition correcte ou de surexposition,
- fourniture de l'état d'indication du niveau d'exposition utilisé, dans lequel le traitement du signal de sortie comprend:
- la détermination (S2) dans le signal numérique des valeurs d'amplitude extrêmes,
- une première étape (S4) de comparaison d'une différence (Δ) entre la valeur d'amplitude maximum et la valeur d'amplitude minimum à une première valeur de seuil 1, permettant ainsi d'indiquer si le niveau d'exposition correspond à une sous-exposition ou non, puis,
- une deuxième étape (S6) de comparaison de la valeur d'amplitude maximum, à une deuxième valeur seuil 2 proche du bord de la fenêtre du convertisseur, lorsque la différence (Δ) est supérieure à la première valeur de seuil 1, permettant ainsi d'indiquer si le niveau d'exposition correspond à une exposition correcte ou non.

2. Procédé selon la revendication 1, **caractérisé en ce que** le traitement comprend préalablement une étape de formation d'une courbe traduisant le nombre de pixels du signal d'image par niveau de gris.

3. Procédé selon la revendication 2, **caractérisé en ce que** le traitement comprend une étape de détermination des valeurs d'amplitude extrêmes (max, min) des niveaux de gris numériques de la courbe.

4. Procédé selon la revendication 1, **caractérisé en ce que** le traitement comprend une étape de décision quant à la fourniture de l'état d'indication du niveau d'exposition utilisé ou à la réalisation d'une deuxième étape de comparaison en fonction du résultat de la première étape de comparaison.

5. Procédé selon la revendication 1, **caractérisé en ce que** l'état d'indication du niveau d'exposition utilisé correspond à un niveau de sous-exposition lorsque la différence Δ est inférieure ou égale à la première valeur seuil 1.

6. Procédé selon la revendication 1, **caractérisé en ce que** le traitement comprend une étape de décision quant à la fourniture de l'état d'indication du niveau d'exposition utilisé ou à la réalisation d'un test additionnel, en fonction du résultat de la deuxième étape de comparaison.

7. Procédé selon la revendication 1, **caractérisé en ce que** l'état d'indication du niveau d'exposition utilisé correspond à un niveau d'exposition correct lorsque la valeur max est inférieure à la deuxième valeur seuil 2.

8. Procédé selon la revendication 1, **caractérisé en ce que** lorsque la valeur max est supérieure ou égale à la deuxième valeur seuil 2, alors le traitement comprend une étape de test additionnel afin de déterminer la présence ou:l'absence de données indicatives d'un débordement dans le signal de sortie numérique.

9. Procédé selon la revendication 8, **caractérisé en ce que** en fonction de la détermination de la présence ou de l'absence de données indicatives d'un débordement, le traitement comporte une étape de décision quant à la fourniture de l'état d'indication du niveau d'exposition utilisé correspondant respectivement à un niveau de surexposition ou à un niveau d'exposition correct.

10. Procédé selon la revendication 1, **caractérisé en ce qu'**il comporte une étape d'affichage de l'état d'indication du niveau d'exposition utilisé sur un écran de visualisation.

11. Procédé selon la revendication 10, **caractérisé en ce qu'**il comporte une étape d'affichage du signal d'image correspondant sur l'écran de visualisation.

12. Procédé selon la revendication 10, **caractérisé en ce que** l'état d'indication du niveau d'exposition est affiché sous la forme d'au moins un indicateur dont la position varie en fonction de l'état obtenu par le traitement du signal.

13. Procédé selon la revendication 10, **caractérisé en ce que** l'état d'indication du niveau de sous-exposition, d'exposition correcte ou de surexposition est affiché sous la forme d'un curseur produisant un effet visuel qui varie au moins pour certains niveaux d'exposition.

14. Procédé selon la revendication 13, **caractérisé en ce que** l'effet visuel est la couleur.

15. Procédé selon la revendication 1, **caractérisé en ce que** le convertisseur analogique numérique a une fenêtre d'entrée adaptée à la dynamique du signal analogique délivré par le capteur.

16. Appareil de radiologie dentaire comprenant un capteur intrabuccal qui est adapté à délivrer au moins un signal de sortie analogique d'image en réponse à une exposition dudit capteur à un rayonnement X, comportant
- une unité de conversion dudit au moins un signal de sortie analogique d'image en un signal de sortie numérique d'image, l'unité de conversion ayant une fenêtre de sortie,
- une unité de traitement du signal de sortie numérique d'image qui est adaptée à obtenir un état d'indication du niveau d'exposition au rayonnement X qui a été utilisé pour délivrer ledit signal de sortie analogique, l'état d'indication du niveau d'exposition étant qualifié de sous-exposition, d'exposition correcte ou de surexposition,
- des moyens de fourniture de l'état d'indication du niveau d'exposition utilisé,
- des moyens de détermination dans le signal numérique des valeurs d'amplitude extrêmes,
- des moyens de détermination d'une différence Δ = max-min entre la valeur d'amplitude maximale max et la valeur d'amplitude minimale min.
- des premiers moyens de comparaison de la différence Δ à une première valeur seuil 1, permettant ainsi d'indiquer si le niveau d'exposition correspond à une sous-exposition ou non.
- des deuxièmes moyens de comparaison de la valeur max à une deuxième valeur seuil 2 proche du bord de la fenêtre de l'unité de conversion et qui sont adaptés à être mis en oeuvre lorsque la différence Δ est supérieure à la première valeur seuil 1, permettant ainsi d'indiquer si le niveau d'exposition correspond à une exposition correcte ou non.

17. Appareil selon la revendication 16, **caractérisé en ce que** l'unité de traitement comprend des moyens de formation d'une courbe traduisant le nombre de pixels du signal d'image par niveau de gris.

18. Appareil selon la revendication 17, **caractérisé en ce que** l'unité de traitement comprend des moyens de détermination des valeurs d'amplitude extrêmes (max, min) des niveaux de gris numériques de la courbe.

19. Appareil selon la revendication 17, **caractérisé en ce que** l'unité de traitement comprend des moyens de décision quant à la fourniture de l'état d'indication du niveau d'exposition utilisé ou à la réalisation d'une deuxième comparaison en fonction du résultat fourni par les premiers moyens de comparaison.

20. Appareil selon la revendication 16, **caractérisé en ce que** l'unité de traitement comprend des moyens de décision quant à la fourniture de l'état d'indication du niveau d'exposition utilisé ou à la réalisation d'un test additionnel, en fonction du résultat fourni par les deuxièmes moyens de comparaison.

21. Appareil selon la revendication 16, **caractérisé en ce que** l'unité de traitement comprend des moyens de détermination de la présence ou de l'absence de données indicatives d'un débordement dans le signal de sortie numérique qui sont adaptés à être mis en oeuvre lorsque la valeur max est supérieure ou égale à la deuxième valeur seuil 2.

22. Appareil selon la revendication 16, **caractérisé en ce que** l'unité de traitement comporte des moyens de décision quant à la fourniture de l'état d'indication du niveau d'exposition utilisé correspondant respectivement à un niveau de surexposition ou à un niveau d'exposition correct, en fonction de la détermination de la présence ou de l'absence de données indicatives d'un débordement.

23. Appareil selon la revendication 16, **caractérisé en ce qu'**il comporte des moyens l'affichage de l'état d'indication du niveau d'exposition utilisé sur un écran de visualisation.

24. Appareil selon la revendication 16, **caractérisé en ce que** l'unité de conversion analogique numérique a une fenêtre d'entrée adaptée à la dynamique du signal analogique délivré par le capteur.

## Claims

1. Signal processing method in a dental radiology apparatus comprising an intrabuccal sensor that delivers at least one analogue image output signal in response to exposure of said sensor to X-rays, including the following steps:
- converting said at least one analogue image output signal into a digital image output signal with a converter having an output window,
- processing the digital image output signal with a view to obtaining a status indicating the level of exposure to X-rays that was used to deliver said analogue output signal, the exposure level indication status corresponding to an exposure level qualified as underexposure, correct exposure or overexposure,
- providing the status indicating the exposure level used,
wherein the processing of the output signal comprises:
- determining (S2) in the digital signal the extreme amplitude values,
- a first step (S4) of comparing a difference (Δ) between the maximum amplitude value and the minimum amplitude value to a first threshold value 1, thereby making it possible to indicate if the exposure level corresponds to underexposure or not, then
- a second step (S6) of comparing the maximum amplitude value to a second threshold value 2 close to the edge of the converter window if the difference (Δ) is greater than the first threshold value 1, thus making it possible to indicate if the exposure level corresponds to correct exposure or not.

2. Method according to claim 1, **characterised in that** the processing comprises beforehand a step of forming a curve reflecting the number of pixels of the image signal per grey level.

3. Method according to claim 2, **characterised in that** the processing comprises a step of determining extreme amplitude values (max, min) of the digital grey levels of the curve.

4. Method according to claim 1, **characterised in that** the processing comprises a step of deciding to provide the status indicating the exposure level used or to execute a second comparison step as a function of the result of the first comparison step.

5. Method according to claim 1, **characterised in that** the status indicating the exposure level used corresponds to an underexposure level if the difference Δ is less than or equal to the first threshold value 1.

6. Method according to claim 1, **characterised in that** the processing comprises a step of deciding to provide the status indicating the exposure level used or to execute an additional test as a function of the result of the second comparison step.

7. Method according to claim 1, **characterised in that** the status indicating the exposure level used corresponds to a correct exposure level if the value max is less than the second threshold value 2.

8. Method according to claim 1, **characterised in that** if the value max is greater than or equal to the second threshold value 2 the processing comprises an additional test step in order to determine the presence or the absence of data indicating an overshoot in the digital output signal.

9. Method according to claim 8, **characterised in that** the processing includes a step of deciding to provide the status indicating the exposure level used respectively corresponding to an overexposure level or to a correct exposure level as a function of the determination of the presence or the absence of data indicating an overshoot.

10. Method according to claim 1, **characterised in that** it includes a step of displaying on a display screen the status indicating the exposure level used.

11. Method according to claim 10, **characterised in that** it includes a step of displaying on the display screen the corresponding image signal.

12. Method according to claim 10, **characterised in that** the status indicating the exposure level is displayed in the form of at least one indicator the position of which varies as a function of the status obtained by processing the signal.

13. Method according to claim 10, **characterised in that** the status indicating the underexposure, correct exposure or overexposure level is displayed in the form of a cursor producing a visual effect that varies for at least some exposure levels.

14. Method according to claim 13, **characterised in that** the visual effect is a colour.

15. Method according to claim 1, **characterised in that** the analogue-digital converter has an input window adapted to the dynamic of the analogue signal delivered by the sensor.

16. Dental radiology apparatus comprising an intrabuccal sensor adapted to deliver at least one analogue image output signal in response to exposure of said sensor to X-rays, including:
- a unit for converting said at least one analogue image output signal into a digital image output signal, the conversion unit having an output window,
- a unit for processing said at least one digital image output signal adapted to obtain a status indicating the level of exposure to the X-rays that was used to deliver said analogue output signal, the exposure level of the indication status being qualified as underexposure, correct exposure or overexposure,
- means for providing the status indicating the exposure level used,
- means for determining in the digital signal the extreme amplitude values,
- means for determining a difference Δ = max-min between the maximum amplitude value max and the minimum amplitude value min,
- first means for comparing the difference to a first threshold value 1, thereby making it possible to indicate if the exposure level corresponds to underexposure or not,
- second means for comparing the value max to a second threshold value 2 close to the edge of the converter unit window and adapted to be used if the difference Δ is greater than the first threshold value 1, thus making it possible to indicate if the exposure level corresponds to correct exposure or not.

17. Apparatus according to claim 16, **characterised in that** the processing unit comprises means for forming a curve reflecting the number of pixels of the image signal per grey level.

18. Apparatus according to claim 17, **characterised in that** the processing unit comprises means for determining the extreme amplitude values (max, min) of the digital grey levels of the curve.

19. Apparatus according to claim 17, **characterised in that** the processing unit comprises means for deciding to provide the status indicating the exposure level used or to execute a second comparison as a function of the result supplied by the first comparison means.

20. Apparatus according to claim 16, **characterised in that** the processing unit comprises means for deciding to provide the status indicating the exposure level used or to execute an additional test as a function of the result supplied by the second comparison means.

21. Apparatus according to claim 16, **characterised in that** the processing unit comprises means for determining the presence or the absence of data indicating an overshoot in the digital output signal that are adapted to be used if the value max is greater than or equal to the second threshold value 2.

22. Apparatus according to claim 16, **characterised in that** the processing unit includes means for deciding to provide the status indicating the level of exposure used respectively corresponding to an overexposure level or to a correct exposure level as a function of the determination of the presence or the absence of data indicating an overshoot.

23. Apparatus according to claim 16, **characterised in that** it includes means for displaying on a display screen the status indicating the exposure level used.

24. Apparatus according to claim 16, **characterised in that** the analogue-digital conversion unit has an input window adapted to the dynamic of the analogue signal delivered by the sensor.

## Patentansprüche

1. Verfahren zur Signalverarbeitung in einem Dentalradiologiegerät mit einem intrabukkalen Sensor, der mindestens ein analoges Bildausgangssignal als Antwort auf eine Röntgenbestrahlung des Sensors liefert, das die folgenden Schritte aufweist:
- Umwandlung des mindestens einen analogen Bildausgangssignals in ein digitales Bildausgangssignal durch einen ein Ausgangsfenster aufweisenden Wandler,
- Verarbeitung des digitalen Bildausgangssignals, um einen Anzeigezustand des Röntgenbestrahlungspegels zu erhalten, der verwendet wurde, um das analoge Ausgangssignal zu liefern, wobei der Anzeigezustand des Bestrahlungspegels einem als Unterbestrahlung, korrekte Bestrahlung oder Überbestrahlung bezeichneten Bestrahlungspegel entspricht,
- Lieferung des Anzeigezustands des verwendeten Bestrahlungspegels, bei dem die Verarbeitung des Ausgangssignals enthält:
- die Bestimmung (S2) der extremen Amplitudenwerte im digitalen Signal,
- einen ersten Schritt (S4) des Vergleichs einer Differenz (Δ) zwischen dem maximalen Amplitudenwert und dem minimalen Amplitudenwert mit einem ersten Schwellenwert 1, was es ermöglicht anzuzeigen, ob der Bestrahlungspegel einer Unterbestrahlung entspricht oder nicht, dann
- einen zweiten Schritt (S6) des Vergleichs des maximalen Amplitudenwerts mit einem zweiten Schwellenwert 2 nahe dem Rand des Fensters des Wandlers, wenn die Differenz (Δ) größer ist als der erste Schwellenwert 1, was es ermöglicht anzuzeigen, ob der Bestrahlungspegel einer korrekten Bestrahlung entspricht oder nicht.

2. Verfahren nach Anspruch 1, **dadurch gekennzeichnet, dass** die Verarbeitung vorher einen Schritt der Bildung einer Kurve enthält, die die Anzahl von Pixeln des Bildsignals in Graupegeln ausdrückt.

3. Verfahren nach Anspruch 2, **dadurch gekennzeichnet, dass** die Verarbeitung einen Schritt der Bestimmung der extremen Amplitudenwerte (max, min) der digitalen Graupegel der Kurve enthält.

4. Verfahren nach Anspruch 1, **dadurch gekennzeichnet, dass** die Verarbeitung einen Schritt der Entscheidung bezüglich der Lieferung des Anzeigezustands des verwendeten Bestrahlungspegels oder der Durchführung eines zweiten Vergleichsschritts in Abhängigkeit vom Ergebnis des ersten Vergleichsschritts enthält.

5. Verfahren nach Anspruch 1, **dadurch gekennzeichnet, dass** der Anzeigezustand des verwendeten Bestrahlungspegels einem Unterbestrahlungspegel entspricht, wenn die Differenz Δ geringer als der oder gleich dem ersten Schwellenwert 1 ist.

6. Verfahren nach Anspruch 1, **dadurch gekennzeichnet, dass** die Verarbeitung einen Schritt der Entscheidung bezüglich der Lieferung des Anzeigezustands des verwendeten Bestrahlungspegels oder der Durchführung eines zusätzlichen Tests in Abhängigkeit vom Ergebnis des zweiten Vergleichsschritts enthält.

7. Verfahren nach Anspruch 1, **dadurch gekennzeichnet, dass** der Anzeigezustand des verwendeten Bestrahlungspegels einem korrekten Bestrahlungspegel entspricht, wenn der Wert max geringer als der zweite Schwellenwert 2 ist.

8. Verfahren nach Anspruch 1, **dadurch gekennzeichnet, dass**, wenn der Wert max größer als der oder gleich dem zweiten Schwellenwert 2 ist, die Verarbeitung einen zusätzlichen Testschritt enthält, um die Anwesenheit oder die Abwesenheit von Daten festzustellen, die einen Überlauf im digitalen Ausgangssignal anzeigen.

9. Verfahren nach Anspruch 8, **dadurch gekennzeichnet, dass**, in Abhängigkeit vom Feststellen der Anwesenheit oder Abwesenheit von einen Überlauf anzeigenden Daten, die Verarbeitung einen Schritt der Entscheidung bezüglich der Lieferung des Anzeigezustands des verwendeten Bestrahlungspegels entsprechend einem Überbestrahlungspegel bzw. einem korrekten Bestrahlungspegel enthält.

10. Verfahren nach Anspruch 1, **dadurch gekennzeichnet, dass** es einen Schritt der Anzeige des Anzeigezustands des verwendeten Bestrahlungspegels auf einem Anzeigebildschirm aufweist.

11. Verfahren nach Anspruch 10, **dadurch gekennzeichnet, dass** es einen Schritt der Anzeige des entsprechenden Bildsignals auf dem Anzeigebildschirm aufweist.

12. Verfahren nach Anspruch 10, **dadurch gekennzeichnet, dass** der Anzeigezustand des Bestrahlungspegels in Form mindestens eines Anzeigers angezeigt wird, dessen Position in Abhängigkeit vom durch die Verarbeitung des Signals erhaltenen Zustand variiert.

13. Verfahren nach Anspruch 10, **dadurch gekennzeichnet, dass** der Anzeigezustand des Pegels der Unterbestrahlung, der korrekten Bestrahlung oder der Überbestrahlung in Form eines Cursors angezeigt wird, der eine visuelle Wirkung erzeugt, die mindestens für bestimmte Bestrahlungspegel variiert.

14. Verfahren nach Anspruch 13, **dadurch gekennzeichnet, dass** die visuelle Wirkung Farbe ist.

15. Verfahren nach Anspruch 1, **dadurch gekennzeichnet, dass** der Analog-Digital-Wandler ein Eingangsfenster hat, das an die Dynamik des vom Sensor gelieferten analogen Signals angepasst ist.

16. Dentalradiologiegerät, das einen intrabukkalen Sensor enthält, der geeignet ist, um mindestens ein analoges Bildausgangssignal als Antwort auf eine Röntgenbestrahlung des Sensors zu liefern, das aufweist:
- eine Einheit zur Umwandlung des mindestens einen analogen Bildausgangssignals in ein digitales Bildausgangssignal, wobei die Umwandlungseinheit ein Ausgangsfenster hat,
- eine Einheit zur Verarbeitung des digitalen Bildausgangssignals, die geeignet ist, um einen Anzeigezustand des Röntgenbestrahlungspegels zu erhalten, der verwendet wurde, um das analoge Ausgangssignal zu liefern, wobei der Anzeigezustand des Bestrahlungspegels als Unterbestrahlung, korrekte Bestrahlung oder Überbestrahlung bezeichnet wird,
- Einrichtungen zur Lieferung des Anzeigezustands des verwendeten Bestrahlungspegels,
- Einrichtungen zur Bestimmung der extremen Amplitudenwerte im digitalen Signal,
- Einrichtungen zur Bestimmung einer Differenz Δ = max-min zwischen dem maximalen Amplitudenwert max und dem minimalen Amplitudenwert min,
- erste Einrichtungen zum Vergleich der Differenz Δ mit einem ersten Schwellenwert 1, wodurch es ermöglicht wird anzuzeigen, ob der Bestrahlungspegel einer Unterbestrahlung entspricht oder nicht,
- zweite Einrichtungen zum Vergleich des Werts max mit einem zweiten Schwellenwert 2 nahe dem Rand des Fensters der Umwandlungseinheit, und die geeignet sind, um eingesetzt zu werden, wenn die Differenz Δ höher ist als der erste Schwellenwert 1, wodurch es ermöglicht wird anzuzeigen, ob der Bestrahlungspegel einer korrekten Bestrahlung entspricht oder nicht.

17. Gerät nach Anspruch 16, **dadurch gekennzeichnet, dass** die Verarbeitungseinheit Einrichtungen zur Bildung einer Kurve enthält, die die Anzahl von Pixeln des Bildsignals durch Graupegel ausdrückt.

18. Gerät nach Anspruch 17, **dadurch gekennzeichnet, dass** die Verarbeitungseinheit Einrichtungen zur Bestimmung der extremen Amplitudenwerte (max, min) der digitalen Graupegel der Kurve enthält.

19. Gerät nach Anspruch 17, **dadurch gekennzeichnet, dass** die Verarbeitungseinheit Einrichtungen zur Entscheidung bezüglich der Lieferung des Anzeigezustands des verwendeten Bestrahlungspegels oder der Durchführung eines zweiten Vergleichs in Abhängigkeit von dem von den ersten Vergleichseinrichtungen gelieferten Ergebnis enthält.

20. Gerät nach Anspruch 16, **dadurch gekennzeichnet, dass** die Verarbeitungseinheit Einrichtungen zur Entscheidung bezüglich der Lieferung des Anzeigezustands des verwendeten Bestrahlungspegels oder der Durchführung eines zusätzlichen Tests in Abhängigkeit von dem von den zweiten Vergleichseinrichtungen gelieferten Ergebnis enthält.

21. Gerät nach Anspruch 16, **dadurch gekennzeichnet, dass** die Verarbeitungseinheit Einrichtungen zur Bestimmung der Anwesenheit oder Abwesenheit von einen Überlauf im digitalen Ausgangssignal anzeigenden Daten enthält, die geeignet sind, um eingesetzt zu werden, wenn der Wert max höher als der oder gleich dem zweiten Schwellenwert 2 ist.

22. Gerät nach Anspruch 16, **dadurch gekennzeichnet, dass**, in Abhängigkeit vom Feststellen der Anwesenheit oder der Abwesenheit von einen Überlauf anzeigenden Daten, die Verarbeitungseinheit Einrichtungen zur Entscheidung bezüglich der Lieferung des Anzeigezustands des verwendeten Bestrahlungspegels entsprechend einem Überbestrahlungspegel bzw. einem korrekten Bestrahlungspegel enthält.

23. Gerät nach Anspruch 16, **dadurch gekennzeichnet, dass** es Einrichtungen zur Anzeige des Anzeigezustands des verwendeten Bestrahlungspegels auf einem Anzeigebildschirm enthält.

24. Gerät nach Anspruch 16, **dadurch gekennzeichnet, dass** die Analog-Digital-Umwandlungseinheit ein Eingangsfenster hat, das an die Dynamik des vom Sensor gelieferten analogen Signals angepasst ist.
